(19) **Europäisches Patentamt / European Patent Office / Office européen des brevets**

(11) **EP 1 837 035 A1**

(12) # EUROPEAN PATENT APPLICATION
published in accordance with Art. 158(3) EPC

(43) Date of publication:
**26.09.2007 Bulletin 2007/39**

(21) Application number: **06700849.0**

(22) Date of filing: **12.01.2006**

(51) Int Cl.:
*A61K 45/00* (2006.01)  *A61K 9/14* (2006.01)
*A61K 9/72* (2006.01)  *A61K 31/7088* (2006.01)
*A61K 31/713* (2006.01)  *A61K 48/00* (2006.01)
*A61P 11/00* (2006.01)  *C12N 15/09* (2006.01)

(86) International application number:
**PCT/JP2006/300665**

(87) International publication number:
**WO 2006/075776 (20.07.2006 Gazette 2006/29)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.01.2005 JP 2005006893**

(71) Applicant: **AnGes MG, Inc.**
**Ibaraki-shi,**
**Osaka 567-0085 (JP)**

(72) Inventors:
- **OTSU, Kunimiki**
  **Tokyo 195-0071 (JP)**
- **SAITO, Tetsu**
  **Shizuoka 411-0020 (JP)**
- **GEMBA, Takefumi**
  **Hyogo 666-0014 (JP)**

(74) Representative: **HOFFMANN EITLE**
**Patent- und Rechtsanwälte**
**Arabellastrasse 4**
**81925 München (DE)**

(54) **THERAPEUTIC AGENT FOR CHRONIC OBSTRUCTIVE PULMONARY DISEASE (COPD), CYSTIC FIBROSIS OR PULMONARY HYPERTENSION**

(57)  The present invention provides a prophylactic, therapeutic or ameliorative medicament for chronic obstructive pulmonary disease (COPD), cystic fibrosis or pulmonary hypertension. More specifically, the present invention provides: (1) a prophylactic, therapeutic or ameliorative medicament for chronic obstructive pulmonary disorder (COPD), cystic fibrosis or pulmonary hypertension, having an NF-κB decoy as the active ingredient thereof; (2) the NF-κB decoy is an oligonucleotide containing the binding sequence GGGRHTYYC (where R represents either A or G; Y represents either C or T; and H represents either A, C or T); (3) an oligonucleotide wherein the NF-κB binding sequence is GGGATTTCCC or GGGACTTTCC; (4) an oligonucleotide wherein the NF-κB decoy is represented by SEQ ID NO:3; (5) the NF-κB decoy is a double-stranded oligonucleotide; (6) the NF-κB decoy is administered in the form of a fine powder; and (7) the NF-κB decoy fine powder is a dry powder.

EP 1 837 035 A1

SEQUENCE LISTING

<110> Anges MG, Inc

<120> Therapeutic agent for COPD, cystic fibrosis or pulmonary hypertension

<130> 05118PCT

<160> 3

<210> 1
<211> 10
<212> DNA
<213> Artificial Sequence

<220>
<223> NF-κB consensus sequence

<400> 1
gggrhtyyhc                                            10


<210> 2
<211> 10
<212> DNA
<213> Artificial Sequence

<220>
<223> NF-κB binding sequence

<400> 2
gggatttccc                                            10


<210> 3
<211> 20
<212> DNA
<213> Artificial Sequence

<220>
<223> NF-κB decoy

<400> 3
ccttgaaggg atttccctcc                                 20


<210> 4
<211> 10
<212> DNA
<213> Artificial Sequence

<220>
<223> NF-κB binding sequence

<400> 4
gggactttcc                                            10

**Description**

Technical Field

The present invention provides a prophylactic, therapeutic or ameliorative medicament containing an NF-κB decoy as an active ingredient for chronic obstructive pulmonary disease (COPD), cystic fibrosis or pulmonary hypertension.

Background Art

COPD is a disease characterized by an obstructive ventilatory impairment of the lungs. The bronchial epithelial cells become damaged by chronic smoking and the like, and this damage triggers the infiltration of inflammatory cells, causing inflammation in the bronchi and alveoli. The secretion of mucus increases, and cough and expectoration of sputum appear as early symptoms of COPD. As COPD progresses further, thickening of the airway walls and destruction of alveolar supporting tissue advance due to inflammation, and a decrease in the ratio of forced expiratory volume per second (FEV1) occurs due to obstructive pulmonary dysfunction and loss of elastic lung recoil. Generally speaking, this is a disease with a long clinical course and poor prognosis. It has been estimated that the number of latent COPD patients in Japan is at least 5 million, and it is believed that the number of patients will increase in the future as aging of population. At present, bronchodilators and glucocorticoids are used for the management of COPD (Reference 4), but no basic mode of therapy exists. In addition, it has been pointed out that the risk of osteoporosis increases with the use of inhaled steroids (Reference 13).

Cystic fibrosis (CF), on the other hand, is a hereditary disease, and CFTR (cystic fibrosis transmembrane conductance regulator) has been revealed as the causative gene thereof. There are at least 50,000 patients suffering from this disease. CFTR forms chloride ion channels, and the ability to transport chloride ions out of the cell is lost due to mutations in this gene; as a result, it is believed that damage occurs to bronchial and alveolar epithelial cells as a result of dehydration of the intrabronchial lumina (Reference 4). Just as in the case of COPD, a pattern of inflammation appears due to infiltration by inflammatory cells, bronchial and alveolar obstruction occurs due to thick mucus, and ventilatory impairment progresses. Patients with COPD or CF are vulnerable to infection which exacerbates the disease.

Pulmonary hypertension occurs in the late stage of COPD (stage III: advanced COPD); normally it appears after severe hypoxemia acidosis ($PaO_2 < 8.0$ kPa or 60 mmHg), and it is often accompanied by hypercapnia. Pulmonary hypertension is the main cardiovascular complication of COPD, and it is linked to pulmonary heart disease. The prognosis of pulmonary hypertension is poor. In patients with advanced COPD, however, the pulmonary arterial pressure is only slightly higher than normal under resting conditions, but increases markedly with physical exertion. Even if pulmonary hypertension associated with COPD remains untreated, its progression is slow.

Both COPD and CF are obstructive pulmonary diseases accompanied by metaplasia of the goblet cells that secrete mucus and inflammation accompanied by mucus plugs; they also have in common the fact that the inflammatory cells involved therein are mainly monocytes/macrophages and neutrophils (Reference 5, 8 and 10). Because recovery of the destroyed bronchial and alveolar epithelial cells is almost impossible, it is important in the treatment of these diseases to effectively suppress the inflammatory response to prevent further destruction of cells.

NF-κB is a factor that regulates the expression of various cytokine, chemokine, and cell adhesion factor genes, and it is already known from atopic and rheumatoid arthritis animal models that when the action of NF-κB is inhibited, various inflammatory and immunological responses are suppressed. With respect to obstructive pulmonary diseases, it has also been reported that NF-κB activation is caused by tobacco smoke in a mouse model (Reference 6).

Reference List of Relevant Techniques

1) Marcus Mall et al. Increased airway epithelial Na+ absorption produces cystic fibrosis-like lung disease in mice. Nature Medicine 10: (5) 487-493 (2004).
2) Shao MX, Nakanaga T, Nadel JA. Cigarette smoke induces MUC5AC mucin overproduction via tumor necrosis factor-alpha-converting enzyme in human airway epithelial (NCI-H292) cells. Am J Physiol Lung Cell Mol Physiol. 287 (2): L420-427 (2004).
3) Takeyama K, Birgit J, et al. Activation of epidermal growth factors is responsible for mucin synthesis induced by cigarette smoke. Am J Physiol Lung Cell Mol Physiol 280: L165-L172, (2001).
4) Matsui H. et al. Evidence for periciliary liquid layer depletion, not abnormal ion composition, in the pathogenesis of cystic fibrosis airways disease. Cell 95: 1005-1015 (1998).
5) Chung KF. Cytokines in chronic obstructive pulmonary disease. Eur Respir J Suppl 34:50s-59s. Review (2001).

6) Churg A, Wang RD, Tai H, Wang X, Xie C, Dai J, Shapiro SD, Wright JL. Macrophage metalloelastase mediates acute cigarette smoke-induced inflammation via tumor necrosis factor-alpha release. Am J Respir Crit Care Med. 167 (8): 1045-1046 (2003) .

7) Desmet C, et al. Selective blockade of NF-kB activity in air way immune cells inhibits the effector phase of experimental asthma. J. Immunology 173; 5766-5775 (2004).

8) Singh S, Syme CA, Singh AK, Devor DC, Bridges RJ. Benzimidazolone activators of chloride secretion: potential therapeutics for cystic fibrosis and chronic obstructive pulmonary disease. J Pharmacol Exp Ther. 296(2):600-611 (2001) .

9) Sin DD, Johnson M, Gan WQ, Man SF. Combination therapy of inhaled corticosteroids and long-acting beta2-aderenergics in management of patients with chronic obstructive pulmonary disease. Curr Pharm Des. 10: (28) 3547-3560 (2004).

10) Hubbard RC, Fells G, Gadek J, Pacholok S, Humes J, Crystal RG. Neutrophil accumulation in the lung in alpha 1-antitrypsin deficiency. Spontaneous release of leukotriene B4 by alveolar macrophages. J Clin Invest. 88: (3): 891-897 (1991).

11) Dahl M, Tybjaerg-Hansen A, Lange P, Nordestgaard BG. DeltaF508 heterozygosity in cystic fibrosis and susceptibility to asthma. Lancet. 1998 Jun 27;351(9120):1911-3.

12) Sin DD, Johnson M, Gan WQ, Man SF. Combination therapy of inhaled corticosteroids and long-acting beta2-adrenergics in management of patients with chronic obstructive pulmonary disease. Curr Pharm Des 10:3547-3560 (2004) .

13) Scanlon PD, Connett JE, Wise RA, Tashkin DP, Madhok T, Skeans M, Carpenter PC, Bailey WC, Buist AS, Eichenhorn M, Kanner RE, Weinmann G. Loss of Bone Density with Inhaled Triamcinolone in Lung Health Study II. Am J Respir Crit Care Med. 170(12):1302-1309 (2004).

WO-A 03/105780 discloses a method wherein an antisense dry powder is administered for the treatment of various diseases.

Disclosure of the invention

WO-A 03/105780 makes no disclosure whatsoever of the use of an NF-κB decoy.

In addition, the kinds of effects brought about by the inhibition of NF-κB action are not sufficiently revealed therein. The inventors have completed the inventions of the present application by demonstrating that administration of a DNA derivative of approximately 20 bases in length (NF-κB decoy) containing the DNA sequence to which NF-κB binds when regulating gene expression (consensus sequence) is effective at low doses in a COPD guinea pig model.

In English the term decoy refers a kind of "lure", and a substance having a structure similar to the structure of a genuine substance to be bound or affected by a certain substance is also called a decoy. As a decoy for a transcription factor binding to the binding region on a genomic gene, a double-stranded oligonucleotide having the same base sequence as the binding region is mainly used.

In the presence of a decoy comprising such an oligonucleotide, some of the transcription factor molecules will bind to the oligonucleotide decoy without binding to the binding region of the genomic gene to which it is originally supposed to bind. Thus, the number of molecules of transcription factor binding to the binding region on the genomic gene to which it is actually supposed to bind decreases, and this results in a decrease in the transcription factor activity. In such a case, the oligonucleotide is called a decoy because it binds to the transcription factor and functions as a counterfeit version (lure) of the binding region of the genuine genomic gene. In addition, the term consensus sequence refers to a shared gene sequence to which a given transcription factor binds. For example, the consensus sequence to which NF-κB binds is GGGRHTYYHC (wherein R represents either A or G; Y represents either C or T; and H represents either A, C or T) (SEQ ID No:1). In the case of NF-κB, it is possible to demonstrate sequences gggatttccc (SEQ ID No:2) or gggactttcc (SEQ ID No:4) and the like on the basis of the aforementioned consensus sequence.

The present invention provides (1) a prophylactic, therapeutic or ameliorative medicament for chronic obstructive pulmonary disease (COPD), cystic fibrosis, or pulmonary hypertension, having the aforementioned NF-κB decoy as an active ingredient thereof.

The present invention provides a prophylactic, therapeutic or ameliorative method for chronic obstructive pulmonary disease (COPD), cystic fibrosis or pulmonary hypertension, wherein the aforementioned NF-κB decoy is administered to a patient at a pharmacologically effective dose.

The present invention provides a use of the aforementioned NF-κB decoy for manufacturing a prophylactic, therapeutic or ameliorative medicament for chronic obstructive pulmonary disease (COPD), cystic fibrosis or pulmonary hypertension.

Detailed description of the invention

The present invention contains the following as preferred embodiments.

(2) The prophylactic, therapeutic or ameliorative medicament according to (1), wherein the NF-κB decoy is an oligonucleotide containing the binding sequence GGGRHTYYHC (where R represents either A or G; Y represents either C or T; and H represents either A, C or T).

(3) The prophylactic, therapeutic or ameliorative medicament according to (1) or (2), wherein the aforementioned NF-κB binding sequence is GGGATTTCCC or GGGACTTTCC.

(4) The prophylactic, therapeutic or ameliorative medicament according to any of (1) to (3), wherein the NF-κB decoy is the oligonucleotide represented by SEQ ID NO:3.

(5) The prophylactic, therapeutic or ameliorative medicament according to any of (1) to (4), wherein the NF-κB decoy is a double-stranded oligonucleotide.

(6) The prophylactic, therapeutic or ameliorative medicament according to any of (1) to (5), wherein the NF-κB decoy comprises the oligonucleotide represented by SEQ ID NO: 3 and an oligonucleotide having a sequence that is perfectly complementary thereto.

(7) The prophylactic, therapeutic or ameliorative medicament according to any of (1) to (6), comprising the NF-κB decoy containing an oligonucleotide in which part or all of the phosphate moieties are phosphothioated as an active ingredient thereof.

(8) The prophylactic, therapeutic or ameliorative medicament according to any of (1) to (7), wherein the NF-κB decoy is administered in the form of a fine powder.

(9) The prophylactic, therapeutic or ameliorative medicament according to any of (1) to (8), wherein the NF-κB decoy fine powder is a dry powder.

(10) The prophylactic, therapeutic or ameliorative medicament according to any of (1) to (9), wherein the mean aerodynamic particle size of the NF-κB decoy fine powder is approximately 0.01 to 50 μm.

(11) The prophylactic, therapeutic or ameliorative medicament according to any of (1) to (10), wherein the mean aerodynamic particle size of the NF-κB decoy fine powder is approximately 0.05 to 30 μm.

(12) The prophylactic, therapeutic or ameliorative medicament according to any of (1) to (11), wherein the aerodynamic mean particle size of the NF-κB decoy fine powder is approximately 0.1 to 10 μm.

Here, the double-stranded oligonucleotide disclosed in US Patent No. 6262033 which comprises the oligonucleotide represented by SEQ ID NO:3 and an oligonucleotide having a sequence that is perfectly complementary thereto, can be listed as a concrete example of the NF-κB decoy of the present invention.

The NF-κB decoy oligonucleotide can be either single-stranded or double-stranded, but the double-stranded form is preferred. In addition, the phosphate moieties can be modified by phosphothioatization and the like.

The method of administration of the NF-κB decoy is not limited, but administration in the form of a fine powder, particularly a dry powder, is preferred. The means of forming the fine powder is not limited, and the fine powders can be manufactured by conventional means, for example, a ball mill, bead mill, jet mill, ultimizer, stone mill, spray drying, supercritical fluid, and the like.

The particle size of the fine powder is not limited, but usually it is approximately 0.01 to 50 μm, preferably 0.05 to 30 μm, and more preferably approximately 0.1 to 10 μm.

When administered in the form of a fine powder, NF-κB decoy is usually formulated as a composition wherein a pharmacologically acceptable carrier is added thereto. The carrier is not limited provided it is used as an excipient, but concrete examples thereof include glucose, lactose, trehalose, sucrose, mannitol, xylitol, and the like.

The method of administration is not limited, and concrete examples thereof include administration using a device such as a metered dose inhaler (MDI), dry powder inhaler (DPI), nebulizer and the like.

Furthermore, the present invention does not restrict the size of the dose, but usually the present invention is administered to an adult as a single dose of approximately 10 μg to 100 mg, preferably approximately 50 μg to 50 mg and more preferably approximately 100 μg to 10 mg.

Brief Description of Drawings

Fig. 1 is a graph comparing airway resistance in groups administered doses of 10 μg/kg, 50 μg/kg and 250 μg/kg of the NF-κB decoy, and in a control (vehicle) group at week 4 of tobacco smoke exposure.

Fig. 2 consists of graphs showing the number of inflammatory cells in bronchoalveolar lavage fluid.

Examples

The present invention is further described in detail below through Examples; needless to say, the present invention is in no way restricted thereto.

Example 1 Effect of NF-κB decoy on guinea pig tobacco smoke COPD model

Model Animals:

Guinea pigs (Hartley, Japan SLC, Inc.) were placed in an exposure holder (RMH-TUBES, Muenster Ltd.), immobilized in an exposure chamber (Flow-past type, nose-only inhalation chamber, Muenster Ltd.), and forced to inhale tobacco smoke (HighLite, Japan Tobacco, Inc.) 60 minutes a day, 5 days a week for 4 weeks using a smoke exposure apparatus (Hamburg II, Borgwaldt Technik) to cause lung damage.

Administered Formulations:

(1) Preparation of NF-κB decoy oligonucleotide fine powder

Approximately 200 mg of NF-κB decoy oligonucleotide comprising a double-stranded oligonucleotide consisting of the oligonucleotide represented by SEQ ID NO:3 and a complementary sequence thereto, and having the phospate bonds thereof phosphotioated was weighed out, placed in a sample tube for a Multi-beads shocker®, and the sample tube was then placed in a metal cone and stoppered. Next, the sample tube was set in the Multi-beads shocker®, and pulverization was performed under the following conditions.
Vibration rate: 2000 rpm
Operating time: 12 cycles of 5 minutes (1 hour) (The sample holder was cooled every 5 minutes using dry ice.)

(2) Preparation of D-mannitol fine powder

Two sample tubes for the Multi-beads shocker® were prepared, approximately 2 g of D-mannitol was weighed out into each, and the tubes were then placed in metal cones and stoppered. Next, the sample tube was set in the Multi-beads shocker®, and pulverization was performed under the following conditions.
Vibration rate: 2000 rpm
Operating time: 12 cycles of 5 minutes (1 hour) (The sample holder was cooled every 5 minutes using dry ice.)
The NF-κB decoy powder and mannitol prepared as fine powders by the above method were accurately weighed out, placed in a sample tube for a Multi-beads shocker®, then the sample tube was placed in a metal cone and stoppered. Pulverization was performed again under the same conditions as mentioned above to prepare a fine powder (mean particle size ≤ 5 μm).

Administration:

Administration was performed endotracheally to the guinea pigs once a week using a dry powder endotracheal dosing device (DP-4).

Measurement of respiratory function:

Respiratory function (specific airway resistance, tidal volume, and minute ventilation volume) of the guinea pigs was measured under anesthesia by double flow plethysmography using a total respiratory function analysis system (Pulmos-I, Kabushiki Kaisha M·I·P·S). Measurements were performed before exposure, and one day after the completion of 2, 3 and 4 weeks of exposure. The respiratory function for 100 respirations was measured in each guinea pig, and the mean value was used as the observed value. The amount of change in respiratory function for each measured week was calculated using the following formula.

Change in respiratory function for each measured week = observed value for respiratory function at each week −

observed respiratory function before tobacco smoke exposure.

Investigation of inflammatory cells in bronchioalveolar lavage fluid:

After pulmonary function was measured, the animals were sacrificed by exsanguination and the thorax was opened. After the division to the left bronchus was ligated, 2.5 mL of physiological saline solution was injected into the right lung and aspirated via the cannula affixed at the time of pulmonary function measurement, the procedure was performed twice (total 5 mL), and the fluid recovered thereby was used as the bronchoalveolar lavage fluid (BALF). Centrifugal separation of the BALF was performed for 10 min at $230 \times g$ (rpm: 1100 rpm, centrifuge radius: 17 cm) at 4°C to obtain a precipitate (pellet). The pellet was suspended in 0.5 mL of physiological saline solution. The pellet was resuspended using micropipettes, added to a 96-well microplate which had been prepared by the addition of Turk solution and diluted 10-fold, and the cell count per 1 $\mu$L was calculated by counting 4 chambers of the large compartment of a hemocytometer (Bürker-Türk hemocytometer: S764).

Test results:

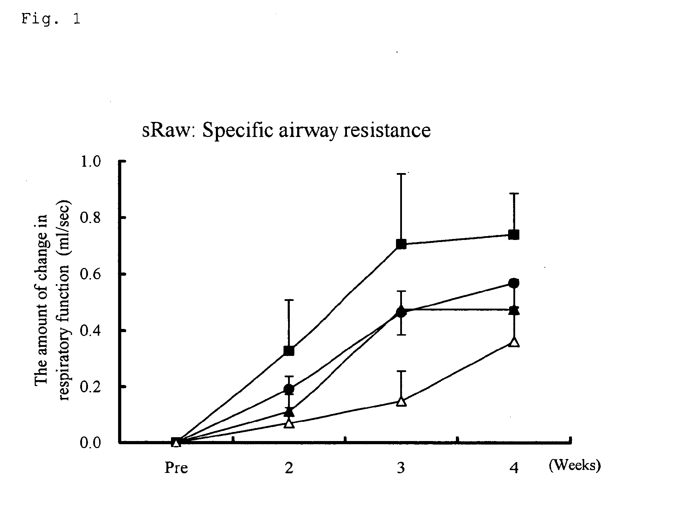

As shown in Fig. 1, the amount of change in airway resistance in the control (vehicle) group at week 4 of tobacco smoke exposure was a mean value of 0.569 mH$_2$O $\times$ mL/(mL/sec), and airway resistance increased over time.

(In Fig. 1, AMG-10, AMG-50 and AMG-250 represent respective doses of 10 $\mu$g/kg, 50 $\mu$g/kg and 250 $\mu$g/kg of NF-$\kappa$B decoy.)

The amount of change in the AMG-10, 50 and 250 dosing groups showed respective mean values of 0.741, 0.475 and 0.360 cm H$_2$O $\times$ mL/(mL/sec) at week 4 of tobacco smoke exposure, and the increase in airway resistance was inhibited in a dose-dependent manner in the NF-$\kappa$B dosing groups.

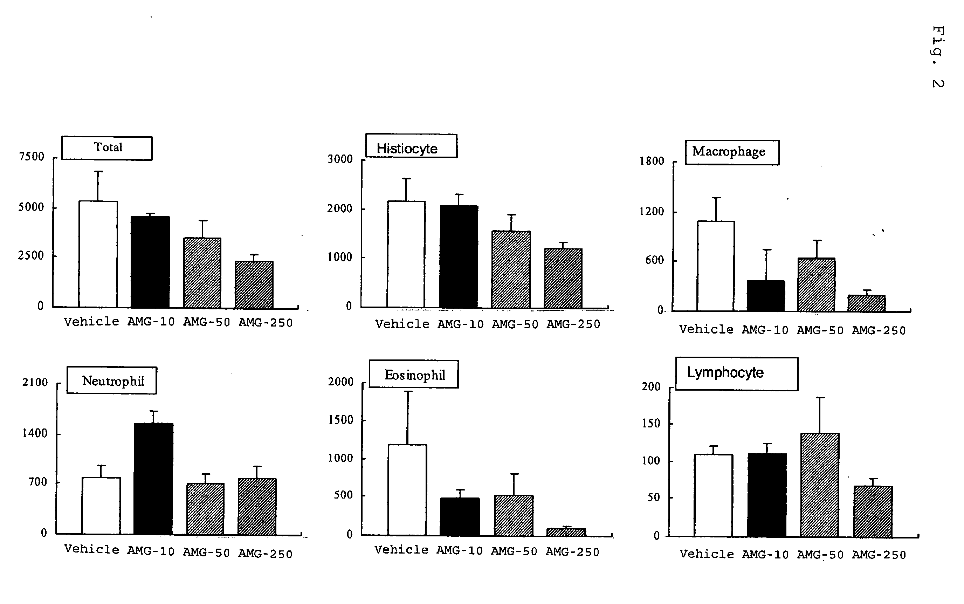

The results of the inflammatory cell count in bronchoalveolar lavage fluid are shown in Figure 2 and the following table.

(Units: Cells/$\mu$L)

| Group | n | Total cells | Histiocytes | Macrophages | Neutrophils | Eosinophils | Lymphocytes |
|---|---|---|---|---|---|---|---|
| Vehicle | 5 | 5365±1476 | 2179±470 | 1097±287 | 781±170 | 1198±680 | 111±11 |
| AMG-10 | 5 | 4605±142 | 2088±245 | 373±81 | 1554±160 | 478±113 | 112±15 |
| AMG-50 | 5 | 3580±833 | 1575±336 | 644±225 | 705±135 | 518±282 | 139±48 |
| AMG-250 | 5 | 2380±313 | 1229±126 | 203±55 | 785±158 | 96±36 | 68±10 |

The mean value for the total number of cells in the bronchoalveolar lavage fluid of the control (vehicle) group exposed to 4 weeks of tobacco smoke was 5365 cells/$\mu$L, and the mean values for histiocytes, macrophages, neutrophils, eosinophils and lymphocytes were 2179, 1097, 781, 1198 and 111 cells/$\mu$L, respectively.

The mean value for the total number of cells in AMG-10 dosing group was 4605 cells/$\mu$L, and the mean values for histiocytes, macrophages, neutrophils, eosinophils and lymphocytes were 2088, 373, 1554, 478 and 112 cells/$\mu$L, respectively; the mean values in AMG-50 dosing group were 3580, 1575, 644, 705, 518 and 139 cells/$\mu$L, respectively; and the mean values in AMG-250 dosing group were 2380, 1229, 203, 785, 96 and 68 cells/$\mu$L, respectively. A decrease in the total number of cells and number of histiocytes in the BALF of the NF-$\kappa$B decoy dosing groups was seen and in particular, a significant suppression of macrophages was seen in the AMG-10 and 250 dosing groups in comparison with the control (vehicle) group. Macrophages also occupy an important position among inflammatory cells involved in COPD, and these results indicate that the NF-$\kappa$B decoy is effective in improving the symptoms of COPD.

**Claims**

1. A prophylactic, therapeutic or ameliorative medicament for chronic obstructive pulmonary disease (COPD), cystic fibrosis, or pulmonary hypertension, comprising an NF-κB decoy as an active ingredient thereof.

2. The prophylactic, therapeutic or ameliorative medicament according to claim 1, wherein the NF-κB decoy is an oligonucleotide containing the binding sequence GGGRHTYYHC (where R represents either A or G; Y represents either C or T; and H represents either A, C or T).

3. The prophylactic, therapeutic or ameliorative medicament according to claim 1 or 2, wherein the NF-κB binding sequence of NF-κ is GGGATTTCCC or GGGACTTTCC.

4. The prophylactic, therapeutic or ameliorative medicament according to any of claims 1 to 3, wherein the NF-κB decoy is the oligonucleotide represented by SEQ ID NO:3.

5. The prophylactic, therapeutic or ameliorative medicament according to any of claims 1 to 4, wherein the NF-κB decoy is a double-stranded oligonucleotide.

6. The prophylactic, therapeutic or ameliorative medicament according to any of claims 1 to 5, wherein the NF-κB decoy comprises the oligonucleotide represented by SEQ ID NO:3 and an oligonucleotide having a sequence that is perfectly complementary thereto.

7. The prophylactic, therapeutic or ameliorative medicament according to any of claims 1 to 6, comprising the NF-κB decoy comprising an oligonucleotide in which part or all of the phosphate moieties are phosphothioated as an active ingredient thereof.

8. The prophylactic, therapeutic or ameliorative medicament according to any of claims 1 to 7, wherein the NF-κB decoy is a fine powder.

9. The prophylactic, therapeutic or ameliorative medicament according to any of claims 1 to 8, wherein the fine powder of NF-κB decoy is a dry powder.

10. The prophylactic, therapeutic or ameliorative medicament according to any of claims 1 to 9, wherein the mean aerodynamic particle size of the fine powder of NF-κB decoy is approximately 0.01 to 50 μm.

11. The prophylactic, therapeutic or ameliorative medicament according to any of claims 1 to 10, wherein the mean aerodynamic particle size of the fine powder of NF-κB decoy is approximately 0.05 to 30 μm.

12. The prophylactic, therapeutic or ameliorative medicament according to any of claims 1 to 11, wherein the mean aerodynamic particle size of the fine powder of NF-κB decoy is approximately 0.1 to 10 μm.

13. A prophylactic, therapeutic or ameliorative method for chronic obstructive pulmonary disease (COPD), cystic fibrosis or pulmonary hypertension, wherein the NF-κB decoy according to claim 1 is administered to a patient at a pharmacologically effective dose.

14. A use of the NF-κB decoy according to any of claims 1 to 12 for manufacturing a prophylactic, therapeutic or ameliorative medicament for chronic obstructive pulmonary disease (COPD), cystic fibrosis or pulmonary hypertension.

Fig. 1

sRaw: Specific airway resistance

Fig. 2

## INTERNATIONAL SEARCH REPORT

| | International application No. |
| --- | --- |
| | PCT/JP2006/300665 |

A.   CLASSIFICATION OF SUBJECT MATTER
*A61K45/00*(2006.01), *A61K9/14*(2006.01), *A61K9/72*(2006.01), *A61K31/7088* (2006.01), *A61K31/713*(2006.01), *A61K48/00*(2006.01), *A61P11/00*(2006.01), *C12N15/09*(2006.01)

According to International Patent Classification (IPC) or to both national classification and IPC

B.   FIELDS SEARCHED

Minimum documentation searched (classification system followed by classification symbols)
A61K9/14, A61K9/72, A61K31/7088, A61K31/713, A61K45/00, A61K48/00, A61P11/00, C12N15/09

Documentation searched other than minimum documentation to the extent that such documents are included in the fields searched
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS(STN), CAplus(STN), EMBASE(STN), MEDLINE(STN)

C.   DOCUMENTS CONSIDERED TO BE RELEVANT

| Category* | Citation of document, with indication, where appropriate, of the relevant passages | Relevant to claim No. |
| --- | --- | --- |
| X<br>Y | GRIESENBACH, U. et al., Anti-inflammatory gene therapy directed at the airwat epithelium, Gene Therapy, Vol.7, 2000, pages 306 to 313 | 1-7<br>1-12,14 |
| Y | WO 1996/035430 A1  (Fujisawa Pharmaceutical Co., Ltd.),<br>14 November, 1996 (14.11.96),<br>Particularly, Claims; page 4, line 23 to page 7, line 3; page 9, lines 16 to 24<br>& JP 2003-128559 A       & EP 824918 A1 | 1-12,14 |
| Y | JP 2002-193813 A  (AnGes MG, Inc.),<br>10 July, 2002 (10.07.02),<br>Particularly, Claims; page 5, right column, lines 3 to 38; page 8, right column, line 35 to page 10, right column, line 15<br>(Family: none) | 1-12,14 |

| ☒   Further documents are listed in the continuation of Box C. | ☐   See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>10 March, 2006 (10.03.06) | Date of mailing of the international search report<br>20 March, 2006 (20.03.06) |
| --- | --- |
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2006/300665 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 9-501057 A  (Isis Pharmaceuticals Inc.), 04 February, 1997 (04.02.97), Particularly, Claims; page 29, line 5 to page 31, line 23 & EP 732941 A1          & WO 1995/12415 A1 | 1-3,5,7-12, 14 |
| X Y | WRIGHT, J.T. et al., The Role of Nuclear Factor Kappa B in the Pathogenesis of Pulmonary Diseases: Implications for Therapy, American Journal of Respiratory Medicine, Vol.2, No.3, 2003, pages 211 to 219 | 1,14 1-12,14 |
| Y | Toru YAMAGUCHI et al., Konnichi no Chiryo Shishin 2004 Nendoban, Vol.46, 2004, pages 1530 to 1535 | 1-12,14 |
| Y | WO 2003/105780 A1  (EPIGENESIS PHARMACEUTICALS, INC.), 24 December, 2003 (24.12.03), Particularly, Claims; page 25, lines 1 to 14; page 34, lines 23 to 32 & US 2004/0092470 A1 | 8-12,14 |
| P,X | JP 2005-160464 A  (Corgentech, Inc.), 23 June, 2005 (23.06.05), & WO 2005/056020 A2 | 1-7 |

Form PCT/ISA/210 (continuation of second sheet) (April 2005)

**INTERNATIONAL SEARCH REPORT**

| International application No. |
| --- |
| PCT/JP2006/300665 |

---

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 13
   because they relate to subject matter not required to be searched by this Authority, namely:
   Claim 13 pertains to methods for treatment of the human body by therapy and thus relates to a subject matter which this International Searching Authority is not required, under the provisions of the PCT Rule 39.1(iv), to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

**Patent documents cited in the description**

- WO 03105780 A

- US 6262033 B

**Non-patent literature cited in the description**

- **MARCUS MALL et al.** Increased airway epithelial Na+ absorption produces cystic fibrosis-like lung disease in mice. *Nature Medicine,* 2004, vol. 10 (5), 487-493
- **SHAO MX ; NAKANAGA T ; NADEL JA.** Cigarette smoke induces MUC5AC mucin overproduction via tumor necrosis factor-alpha-converting enzyme in human airway epithelial (NCI-H292) cells. *Am J Physiol Lung Cell Mol Physiol.,* 2004, vol. 287 (2), L420-427
- **TAKEYAMA K ; BIRGIT J et al.** Activation of epidermal growth factors is responsible for mucin synthesis induced by cigarette smoke. *Am J Physiol Lung Cell Mol Physiol,* 2001, vol. 280, L165-L172
- **MATSUI H. et al.** Evidence for periciliary liquid layer depletion, not abnormal ion composition, in the pathogenesis of cystic fibrosis airways disease. *Cell,* 1998, vol. 95, 1005-1015
- **CHUNG KF.** Cytokines in chronic obstructive pulmonary disease. *Eur Respir J,* 2001, vol. 34, 50s-59s
- **CHURG A ; WANG RD ; TAI H ; WANG X ; XIE C ; DAI J ; SHAPIRO SD ; WRIGHT JL.** Macrophage metalloelastase mediates acute cigarette smoke-induced inflammation via tumor necrosis factor-alpha release. *Am J Respir Crit Care Med.,* 2003, vol. 167 (8), 1045-1046
- **DESMET C et al.** Selective blockade of NF-kB activity in air way immune cells inhibits the effector phase of experimental asthma. *J. Immunology,* 2004, vol. 173, 5766-5775

- **SINGH S ; SYME CA ; SINGH AK ; DEVOR DC ; BRIDGES RJ.** Benzimidazolone activators of chloride secretion: potential therapeutics for cystic fibrosis and chronic obstructive pulmonary disease. *J Pharmacol Exp Ther.,* 2001, vol. 296 (2), 600-611
- **SIN DD ; JOHNSON M ; GAN WQ ; MAN SF.** Combination therapy of inhaled corticosteroids and long-acting beta2-aderenergics in management of patients with chronic obstructive pulmonary disease. *Curr Pharm Des.,* 2004, vol. 10 (28), 3547-3560
- **HUBBARD RC ; FELLS G ; GADEK J ; PACHOLOK S ; HUMES J ; CRYSTAL RG.** Neutrophil accumulation in the lung in alpha 1-antitrypsin deficiency. Spontaneous release of leukotriene B4 by alveolar macrophages. *J Clin Invest.,* 1991, vol. 88 (3), 891-897
- **DAHL M ; TYBJAERG-HANSEN A ; LANGE P ; NORDESTGAARD BG.** DeltaF508 heterozygosity in cystic fibrosis and susceptibility to asthma. *Lancet,* 27 June 1998, vol. 351 (9120), 1911-3
- **SIN DD ; JOHNSON M ; GAN WQ ; MAN SF.** Combination therapy of inhaled corticosteroids and long-acting beta2-adrenergics in management of patients with chronic obstructive pulmonary disease. *Curr Pharm Des,* 2004, vol. 10, 3547-3560
- **SCANLON PD ; CONNETT JE ; WISE RA ; TASHKIN DP ; MADHOK T ; SKEANS M ; CARPENTER PC ; BAILEY WC ; BUIST AS ; EICHENHORN M.** Loss of Bone Density with Inhaled Triamcinolone in Lung Health Study II. *Am J Respir Crit Care Med.,* 2004, vol. 170 (12), 1302-1309